# EUROPEAN PATENT SPECIFICATION

(11) **EP 2 352 846 B1**
(45) Date of publication and mention of the grant of the patent: **07.01.2015**
(21) Application number: 09752181.9
(22) Date of filing: 13.11.2009
(51) Int. Cl.: C12Q 1/68

(54) **RNA DETECTION METHOD**
RNA-NACHWEISVERFAHREN
PROCÉDÉ DE DÉTECTION D'ARN

(30) Priority: 13.11.2008 EP 08019859
(43) Date of publication of application: 10.08.2011
(60) Divisional application: 11193984.9
(73) Proprietor: Riboxx GmbH, 01445 Radebeul (DE)
(72) Inventor: ROHAYEM, Jacques, 01277 Dresden (DE)
(74) Representative: Habermann, Hruschka & Schnabel
(86) International application number: PCT/EP2009/065131
(87) International publication number: WO 2010/055134

(56) References cited:
- EP-A2- 1 318 203
- CHO M W ET AL: "RNA duplex unwinding activity of poliovirus RNA-dependent RNA polymerase 3Dpol." JOURNAL OF VIROLOGY JUN 1993, vol. 67, no. 6, June 1993 (1993-06), pages 3010-3018, XP002563519 ISSN: 0022-538X
- ROHAYEM JACQUES ET AL: "Protein-primed and de novo initiation of RNA synthesis by norovirus 3Dpol." JOURNAL OF VIROLOGY JUL 2006, vol. 80, no. 14, July 2006 (2006-07), pages 7060-7069, XP002575656 ISSN: 0022-538X

## Description

The present invention relates to methods for the detection of target RNA sequences and to RNA amplification methods making use of strand displacement techniques employing RNA-dependent RNA polymerases having RNA-oligonucleotide duplex separation activity and being capable of *de novo* RNA synthesis in the absence of a primer.

The concept of detecting nucleic acid sequences using strand displacement and its employment in PCR amplification reactions has been introduced by Gelfand et al. in the early 1990s (see WO-A-1992/002638). Corresponding methods and kits are commercially available from Roche Diagnostics and are known under the TaqMan technology. The TaqMan principle particularly employs the 5' to 3'-nuclease activity of nucleic acid (in particular DNA) polymerases.

While the TaqMan technology is mainly directed to DNA constructs and, thus, makes use of DNA-dependent DNA polymerases, further studies have shown that also RNA-dependent RNA polymerases have the ability to displace pre-hybridised nucleotide sequences from a template strand when processing from regions downstream to the preformed RNA duplex. For example, Cho et al. (1993), Journal of Virology 67 (6), pages 3010-3018, show that poliovirus RNA-dependent RNA polymerase (RdRp) shows such an RNA duplex separation activity and is capable of displacing a 1000 nt antisense sequence from the template strand. The poliovirus RdRp is, however, a primer-dependent enzyme, i.e. for polymerization of a complementary RNA strand to occur an oligonucleotide primer is required.

Thus, the existing techniques especially for RNA detection and RNA amplification making use of strand displacement have several limitations: the TaqMan approach needs an enzyme having proof reading activity, i. e. 5' to 3'-nuclease activity. Furthermore, at least two oligonucleotides are required: (1) a labelled oligonucleotide for pre-hybridisation to the target sequence and (2) at least one primer for priming the polymerase activity. The latter drawback is also valid for the study according to Cho et al., *supra,* since the poliovirus RdRp is a primer-dependent enzyme as well. The poliovirus RdRp is also capable of initiation of RNA synthesis by back-priming, that occurs after elongation of the 3'-terminus by the RdRp and snapping back of the elongated tail on the template leading through priming to initiation of RNA synthesis. The poliovirus RdRp is not capable of initiation *de novo,* i.e. without priming.

EP 1 318 203 A2 discloses methods for amplification of nucleic acids by primer elongation and displacement of generated transcripts wherein an RNA strand complementary to the target is polymerised by a primer- and DNA-dependent RNA polymerase and the generated transcript is displaced ba a DNA polymerase (DNA- or RNA-dependent).

The technical problem underlying the present invention is therefore to provide novel methods for RNA detection and RNA amplification that have an as low as possible demand of reactants, in particular oligonucleotides, and are particularly useful for the detection and amplification of small RNA species.

The solution to the above technical problem is provided by the embodiments of the present invention as defined in the claims.

In particular, the present invention relates to a method for the detection of a target RNA sequence in a sample comprising the steps of:
(a) contacting single-stranded RNA (ssRNA) molecules present in a sample with a labelled oligonucleotide containing a sequence substantially complementary to a region of the target RNA under hybridisation conditions to provide a mixture of RNA-oligonucleotide duplexes wherein the RNA-oligonucleotide duplexes comprise the target RNA annealed to the labelled oligonucleotide;
(b) maintaining the mixture of step (a) with an RNA-dependent RNA polymerase (RdRp) under RNA polymerisation conditions in the absence of a primer wherein said RdRp has an RNA-oligonucleotide duplex separation activity (or, generally, nucleic acid duplex separation activity) and is capable of synthesising a complementary RNA strand on a single-stranded RNA template without requiring an RNA duplex for initiation of polymerisation such that the RdRp polymerises an RNA strand complementary to the ssRNA in the sample and releases the labelled oligonucleotide; and
(c) detecting and/or measuring the signal generated by the released labelled oligonucleotide.

Further subject matter of the present invention is an RNA amplification method comprising the steps of:
(i) contacting a single-stranded RNA (ssRNA) template with a labelled oligonucleotide containing a sequence substantially complementary to a region of said ssRNA under hybridisation conditions to provide RNA-oligonucleotide duplexes comprising the ssRNA annealed to the labelled oligonucleotide;
(ii) maintaining the RNA-oligonucleotide duplexes of step (i) with an RNA-dependent RNA polymerase (RdRp) under RNA polymerisation conditions in the absence of a primer wherein said RdRp has an RNA-oligonucleotide separation activity (or, generally, nucleic acid duplex separation activity) and is capable of synthesising a complementary RNA strand on a single-stranded RNA template without requiring an RNA duplex for initiation of polymerisation such that the RdRp polymerises an RNA strand complementary to the ssRNA and releases the labelled oligonucleotide;
(iii) separating the RNA duplexes formed by the RdRp between the ssRNA and the polymerised complementary strand;
(iv) optionally, repeating steps (i) to (iii); and
(v) detecting and/or measuring the signal generated by the released labelled oligonucleotide.

The ssRNA template may be provided by any method known in the art such as chemical synthesis, *in vitro* transcription, preparation of total RNA from cells, tissues or other samples such as blood, plasma, liquor etc. dsRNA templates may be separated into ssRNA through heat denaturation.

As used herein, a "sample" refers to any substance containing or presumed to contain RNA and includes a sample of tissue or fluid isolated from an individual or individuals, including but not limited, for example, skin, plasma, serum, spinal fluid, lymph fluid, synovial fluid, urine, tears, blood cells, organs, tumors, and also to samples of *in vitro cell* culture constituents (including, but not limited to, conditioned medium resulting from the growth of cells in cell culture medium, recombinant cells and cell components). The "labelled" oligonucleotide to be used in the methods claimed in the present invention is not necessarily physically derived from any existing or natural sequence but may be generated in any manner, including chemical synthesis, DNA replication, reverse transcription or a combination thereof.

The oligonucleotide according to the present invention is a single-stranded nucleic acid and maybe DNA or RNA. The "oligonucleotide" according to the present invention may also be denoted as "oligoprobe" and may be a polynucleotide of any length. RNA oligonucleotides, i.e. oligriboonucleotides are preferred. The term "oligonucleotide" furthermore intends a polynucleotide of genomic DNA or RNA, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature; and (3) ins not found in nature.

The "oligonucleotide" is further preferred to be a rather small polynucleotide, i.e. preferably it has a length of from 5 to 100, more preferred 5 to 20, most preferred 10 to 12 nucleotides.

The term "label" as used herein refers to any atom or molecule which can be used to provide a detectable (preferably quantifiable) signal, and which can be attached to a nucleic acid, in particular the oligonucleotide. Labels may provide signals detectable by fluorescence, radioactivity, colorimetry, gravimetry, X-ray diffraction or absorption, magnetism, enzymatic activity, and the like. Preferred labels according to the present invention are fluorescent labels.

The single-stranded RNA in the sample and the ssRNA template may as well be of any origin and length. Typical examples of single-stranded RNA species to be detected or amplified by the methods according to the present invention are messenger RNA, viral RNA and the like. Since the present invention is particular applicable to situations in which smaller RNA species are detected and/or amplified, the single-stranded RNA has preferably a length of from 18 to 200, preferably 16 to 40, most preferably 10 to 25 nucleotides. The foregoing lengths apply especially to the RNA amplification method according to the present invention.

Further preferred examples of target/template RNAs that may be detected/amplified by the method according to the invention are microRNA species (also denoted as "miRNA" or "miR"). miRNAs have a role in gene regulation through translational silencing and are encoded by specific genes. Especially in humans, miRNA disruption has been described in association with several cancer forms. Therefore, miRNA profiling has important implications for cancer aetiology, diagnosis and treatment (see, e.g. Esquela-Kerscher et al. (2006) Nat. Rev. Cancer 6, 259-269; Calin et al. (2006) Nat Rev. Cancer 6, 857-866). Using the methods of the invention, it would, for example, be possible to detect specific miRNAs (or disrupted miRNAs) using labelled oligonucleotides specific for such targets/templates.

In particular with respect to detection and/or amplification of RNA species of shorter length, as exemplified above, it is desirable that the single-stranded RNA (either present as a target RNA in a sample in case of the detection method according to the present invention or present as a template RNA in case of the amplification process) contains at least one C at its 3' end, more preferably having an (C)ₙ 3'-terminal repeat (also denoted as "polyC" repeat) with n being integer of at least 2 or 3.

Crucial to the present invention is the activity of special RNA-dependent RNA polymerases capable of displacing oligonucleotides annealed to the target RNA and being furthermore capable of *de novo* RNA synthesis in the absence of any primer.

Enzymes of this category typically have the feature that *de novo* RNA synthesis can be accomplished on an ssRNA strand. Such RNA-dependent RNA polymerases typically show a "right hand conformation" and have a primary sequence comprising a conserved arrangement of the following sequence motives:
a. XXDYS
b. GXPSG
c. YGDD
d. XXYGL
e. XXXXFLXRXX
with the following meanings:
D: aspartate
Y: tyrosine
S: serine
G: glycine
P: proline
L: leucine
F: phenylalanine
R: arginine
X: any amino acid.

The so-called "right hand conformation as used herein means that the tertiary structure (conformation) of the protein folds like a right hand with finger, palm and thumb, as observed in most template-dependent polymerases.

The sequence motif "XXDYS" is the so-called A-motif. The A-motif is responsible for the discrimination between ribonucleosides and deoxyribonucleosides. The motif "GXPSG" is the so-called B-motif. The B-motif is conserved within all representatives of the RdRp family of the corresponding polymerases from *Calicivirdae.* The motif "YGDD" ("C-motif") represents the active site of the enzyme. This motif, in particular the first aspartate residue (in bold, YGDD) plays an important role in the coordination of the metal ions during the Mg²⁺/Mn²⁺-dependent catalysis. The motif "XXYGL" is the so-called D-motif. The D-motif is a feature of template-dependent polymerases. Finally, the "XXXXFLXRXX" motif ("E-motif") is a feature of RNA-dependent RNA polymerases which discriminates them from DNA-dependent RNA polymerases.

Typical representatives of the above types of RdRps are the corresponding enzymes of the calicivirus family *(Caliciviridae).* The RdRps of the calicivirus family are capable of synthesizing complementary strands using as a template any ssRNA template *in vitro,* including heterologous viral, eukaryotic and prokaryotic templates. The ssRNA template may be positive stranded or negative stranded. The RdRp for use in the present invention is capable of synthesizing a complementary strand to the ssRNA to be detected or to be amplified by *de novo* synthesis in the absence of a primer.

The term *"de novo* synthesis in the absence of a primer" in the context of the present invention means that the RdRp is capable of synthesising a complementary RNA strand on a single-stranded RNA template without requiring an RNA duplex (either formed by a separate primer molecule or by back folding of the template) for initiation of polymerisation. For *de novo* RNA synthesis to occur, the RNA target/template should not contain a polyU, polyA or polyG sequence at its 3'-end. Preferably, such *de novo* synthesis occurs on a ssRNA template having at least one C at its 3' end, more preferably having an (C)ₙ 3'-terminal repeat with n being integer of at least 2 or 3.

Especially in cases of detecting or amplifying RNA having a (C)n 3'-terminal repeat, it is preferred that GTP is added in surplus (preferably, 2x 3x, 4x or 5x more) over ATP, UTP and CTP, respectively, in step (b) or (ii) respectively, as defined above.

Preferred embodiments of the RdRp are corresponding enzymes of a human and/or non-human pathogenic calicivirus. Especially preferred is an RdRp of a norovirus, sapovirus, vesivirus or lagovirus, for example the RdRp of the norovirus strain HuCV/NL/Dresden174/1997/GE (GenBank Acc. No. AY741811) or of the sapovirus strain pJG-Sap01 (GenBank Acc. No. AY694184) or an RdRp of the vesivirus strain FCV/Dresden/2006/GE (GenBank Acc. No. DQ424892).

According to especially preferred embodiments of the invention the RdRp is a protein having an amino acid sequence according SEQ ID NO: 1 (norovirus-RdRp), SEQ ID NO: 2 (sapovirus-RdRp) or SEQ ID NO: 3 (vesivirus-RdRp). The person skilled in the art is readily capable of preparing such RdRp, for example by recombinant expression using suitable expression vectors and host organisms (cf. WO-A-2007/012329). To facilitate purification of the RdRp in recombinant expression, it is preferred that the RdRp is expressed with a suitable "tag" (for example GST or (His)₆-tag) at the N- or C-terminus of the corresponding sequence. For example, a histidine tag allows the purification of the protein by affinity chromatography over a nickel or cobalt column in a known fashion. Examples of embodiments of RdRps fused to a histidine tag are the proteins having an amino acid sequence according to SEQ ID NO: 4, SEQ ID NO: 5, SEQ ID NO: 6 or SEQ ID NO: 7. SEQ ID NO: 4 and SEQ ID NO: 5 correspond to a norovirus-RdRp having a histidine tag (SEQ ID NO: 4: C-terminal His-tag; SEQ ID NO: 5: N-terminal His-tag). SEQ ID NO: 6 and SEQ ID NO: 7 correspond to the amino acid sequence of a sapovirus-RdRp having a histidine tag (SEQ ID NO: 6: C-terminal His-tag; SEQ ID NO: 7: N-terminal His-tag). SEQ ID NO: 8 corresponds to the amino acid sequence of vesirius-RdRp having a histidine tag (C-terminal).
SEQ ID NO:1: 
MGGDSKGTYCGAPILGPGSAPKLSTKTKFWRSSTTPLPPGTYEPAYLGGK
DPRVKGGPSLQQVMRDQLKPFTEPRGKPPKPSVLEAAKKTIINVLEQTID
PPEKWSFTQACASLDKTTSSGHPHHMRKNDCWNGESFTGKLADQASKANL
MFEGGKNMTPVYTGALKDELVKTDKIYGKIKKRLLWGSDLATMIRCARAF
GGLMDELKAHCVTLPIRVGMNMNEDGPIIFERHSRYKYHYDADYSRWDST
QQRAVLAAALEIMVKFSSEPHLAQVVAEDLLSPSVVDVGDFKISINEGLP
SGVPCTSQWNSIAHWLLTLCALSEVTNLSPDIIQANSLFSFYGDDEIVST
DIKLDPEKLTAKLKEYGLKPTRPDKTEGPLVISEDLNGLTFLRRTVTRDP
AGWFGKLEQSSILRQMYWTRGPNHEDPSETMIPHSQRPIQLMSLLGEAAL
HGPAFYSKISKLVIAELKEGGMDFYVPRQEPMFRWMRFSDLSTWEGDRNL
APSFVNEDGVEVDKLAAALE
SEQ ID NO: 2: 
MKDEFQWKGLPVVKSGLDVGGMPTGTRYHRSPAWPEEQPGETHAPAPFGAGD
KRYTFSQTEMLVNGLKPYTEPTAGVPPQLLSRAVTHVRSYIETIIGTHRS
PVLTYHQACELLERTTSCGPFVQGLKGDYWDEEQQQYTGVLANHLEQAWD
KANKGIAPRNAYKLALKDELRPIEKNKAGKRRLLWGCDAATTLIATAAFK
AVATRLQVVTPMTPVAVGINMDSVQMQVMNDSLKGGVLYCLDYSKWDSTQ
NPAVTAASLAILERFAEPHPIVSCAIEALSSPAEGYVNDIKFVTRGGLPS
GMPFTSVVNSINHMIYVAAAILQAYESHNVPYTGNVFQVETVHTYGDDCM
YSVCPATASIFHAVLANLTSYGLKPTAADKSDAIKPTNTPVFLKRTFTQT
PHGVRALLDITSITRQFYWLKANRTSDPSSPPAFDRQARSAQLENALAYA
SQHGPVVFDTVRQIAIKTAQGEGLVLVNTNYDQALATYNAWFIGGTVPDP
VGHTEGTHKIVFEME
SEQ ID NO: 3: 
MKVTTQKYDVTKPDISYKGLICKQLDEIRVIPKGTRLHVSPAHTDDYDEC
SHQPASLGSGDPRCPKSLTAIVVDSLKPYCEKTDGPPHDILHRVQRMLID
HLSGFVPMNISSEPSMLAAFHKLNHDTSCGPYLGGRKKDHMIGGEPDKPL
LDLLSSKWKLATQGIGLPHEYTIGLKDELRPVEKVQEGKRRMIWGCDVGV
ATVCAAAFKGVSDAITANHQYGPVQVGINMDGPSVEALYQRIRSAAKVFA
VDYSKWDSTQSPRVSAASIDILRYFSDRSPIVDSAANTLKSPPIAIFNGV
AVKVTSGLPSGMPLTSVINSLNHCLYVGCAILQSLESRNIPVTWNLFSTF
DMMTYGDDGVYMFPMMFASVSDQIFANLTAYGLKPTRVDKSVGAIEPIDP
ESVVFLKRTITRTPHGIRGLLDRGSIIRQFYYIKGENSDDWKTPPKTIDP
TSRGQQLWNACLYASQHGPEFYNKVYRLAEKAVEYEELHFEPPSYHSALE
HYNNQFNGVDTRSDQIDASVMTDLHCDVFEVLE
SEQ ID NO: 4: 
MGGDSKGTYCGAPILGPGSAPKLSTKTKFWRSSTTPLPPGTYEPAYLGGK
DPRVKGGPSLQQVMRDQLKPFTEPRGKPPKPSVLEAAKKTIINVLEQTID
PPEKWSFTQACASLDKTTSSGHPHHMRKNDCWNGESFTGKLADQASKANL
MFEGGKNMTPVYTGALKDELVKTDKIYGKIKKRLLWGSDLATMIRCARAF
GGLMDELKAHCVTLPIRVGMNMNEDGPIIFERHSRYKYHYDADYSRWDST
QQRAVLAAALEIMVKFSSEPHLAQVVAEDLLSPSVVDVGDFKISINEGLP
SGVPCTSQWNSIAHWLLTLCALSEVTNLSPDIIQANSLFSFYGDDEIVST
DIKLDPEKLTAKLKEYGLKPTRPDKTEGPLVISEDLNGLTFLRRTVTRDP
AGWFGKLEQSSILRQMYWTRGPNHEDPSETMIPHSQRPIQLMSLLGEAAL
HGPAFYSKISKLVIAELKEGGMDFYVPRQEPMFRWMRFSDLSTWEGDRNL
APSFVNEDGVEVDKLAAALEHHHHHH
SEQ ID NO: 5: 
MHHHHHHGGDSKGTYCGAPILGPGSAPKLSTKTKFWRSSTTPLPPGTYEPAYLGG
KDPRVKGGPSLQQVMRDQLKPFTEPRGKPPKPSVLEAAKKTIINVLEQTID
PPEKWSFTQACASLDKTTSSGHPHHMRKNDCWNGESFTGKLADQASKANL
MFEGGKNMTPVYTGALKDELVKTDKIYGKIKKRLLWGSDLATMIRCARAF
GGLMDELKAHCVTLPIRVGMNMNEDGPIIFERHSRYKYHYDADYSRWDST
QQRAVLAAALEIMVKFSSEPHLAQVVAEDLLSPSVVDVGDFKISINEGLP
SGVPCTSQWNSIAHWLLTLCALSEVTNLSPDIIQANSLFSFYGDDEIVST
DIKLDPEKLTAKLKEYGLKPTRPDKTEGPLVISEDLNGLTFLRRTVTRDP
AGWFGKLEQSSILRQMYWTRGPNHEDPSETMIPHSQRPIQLMSLLGEAAL
 
HGPAFYSKISKLVIAELKEGGMDFYVPRQEPMFRWMRFSDLSTWEGDRNL
APSFVNEDGVEVDKLAAALE
SEQ ID NO: 6: 
MKDEFQWKGLPVVKSGLDVGGMPTGTRYHRSPAWPEEQPGETHAPAPFGAGD
KRYTFSQTEMLVNGLKPYTEPTAGVPPQLLSRAVTHVRSYIETIIGTHRS
PVLTYHQACELLERTTSCGPFVQGLKGDYWDEEQQQYTGVLANHLEQAWD
KANKGIAPRNAYKLALKDELRPIEKNKAGKRRLLWGCDAATTLIATAAFK
AVATRLQVVTPMTPVAVGINMDSVQMQVMNDSLKGGVLYCLDYSKWDSTQ
NPAVTAASLAILERFAEPHPIVSCAIEALSSPAEGYVNDIKFVTRGGLPS
GMPFTSVVNSINHMIYVAAAILQAYESHNVPYTGNVFQVETVHTYGDDCM
YSVCPATASIFHAVLANLTSYGLKPTAADKSDAIKPTNTPVFLKRTFTQT
PHGVRALLDITSITRQFYWLKANRTSDPSSPPAFDRQARSAQLENALAYA
SQHGPVVFDTVRQIAIKTAQGEGLVLVNTNYDQALATYNAWFIGGTVPDP
VGHTEGTHKIVFEMEHHHHHH
SEQ ID NO: 7: 
MKHHHHHHDEFQWKGLPVVKSGLDVGGMPTGTRYHRSPAWPEEQPGETHA
PAPFGAGDKRYTFSQTEMLVNGLKPYTEPTAGVPPQLLSRAVTHVRSYIE
TIIGTHRSPVLTYHQACELLERTTSCGPFVQGLKGDYWDEEQQQYTGVLA
NHLEQAWDKANKGIAPRNAYKLALKDELRPIEKNKAGKRRLLWGCDAATT
LIATAAFKAVATRLQVVTPMTPVAVGINMDSVQMQVMNDSLKGGVLYCLD
YSKWDSTQNPAVTAASLAILERFAEPHPIVSCAIEALSSPAEGYVNDIKF
VTRGGLPSGMPFTSVVNSINHMIYVAAAILQAYESHNVPYTGNVFQVETV
HTYGDDCMYSVCPATASIFHAVLANLTSYGLKPTAADKSDAIKPTNTPVF
LKRTFTQTPHGVRALLDITSITRQFYWLKANRTSDPSSPPAFDRQARSAQ
LENALAYASQHGPVVFDTVRQIAIKTAQGEGLVLVNTNYDQALATYNAWF
IGGTVPDPVGHTEGTHKIVFEME
SEQ ID NO: 8:

MKVTTQKYDVTKPDISYKGLICKQLDEIRVIPKGTRLHVSPAHTDDYDEC
SHQPASLGSGDPRCPKSLTAIVVDSLKPYCEKTDGPPHDILHRVQRMLID
HLSGFVPMNISSEPSMLAAFHKLNHDTSCGPYLGGRKKDHMIGGEPDKPL
LDLLSSKNKLATQGIGLPHEYTIGLKDELRPVEKVQEGKRRMIWGCDVGV
ATVCAAAFKGVSDAITANHQYGPVQVGINMDGPSVEALYQRIRSAAKVFA
VDYSKWDSTQSPRVSAASIDILRYFSDRSPIVDSAANTLKSPPIAIFNGV
AVKVTSGLPSGMPLTSVINSLNHCLYVGCAILQSLESRNIPVTWNLFSTF
DMMTYGDDGVYMFPMMFASVSDQIFANLTAYGLKPTRVDKSVGAIEPIDP
ESVVFLKRTITRTPHGIRGLLDRGSIIRQFYYIKGENSDDWKTPPKTIDP
TSRGQQLWNACLYASQHGPEFYNKVYRLAEKAVEYEELHFEPPSYHSALE
HYNNQFNGVDTRSDQIDASVMTDLHCDVFEVLEHHHHHH

Another characteristic feature of the enzymes as outlined above, in particular RdRps from *Caliciviridae,* is a terminal transferase activity. This further activity of the preferred enzymes to be used in the methods according to the present invention can successfully be applied for the provision of ssRNA templates with a poly(C) (or poly(U)) terminal repeat at the 3'-end of the ssRNA as outlined above. With respect to the terminal transferase activity of the enzymes according to the present invention, see Rohayem et al. (2006) Journal of General Virology 87, 2621-2630, and the reaction conditions for accessing terminal transferase activity of calicivirus RdRps described therein.

The labelled oligonucleotide used herein is selected to be "substantially" complementary to a region of the target RNA (i. e. the RNA sequence to be detected or to be amplified). Thus, the oligonucleotide needs not to reflect the exact sequence of the target, but must be sufficiently complementary to at least a region of the target for hybridising selectively to it. Non-complementary bases or longer sequences can be interspersed into the oligonucleotide or located at the ends of the oligonucleotide, provided that it retains sufficient complementarity with the template strand to form a stable duplex therewith.

In the present invention, the labelled oligonucleotide must be first annealed to the complementary region of the target ssRNA before polymerization by the RdRp reaches this duplex region. To enhance the likelihood that the labelled oligonucleotide will have annealed to the complementary region of the target before the RdRp reaches the duplex region, a variety of techniques can be employed. For example, one can position the oligonucleotide so that its 5'-end is relatively far from the 3'-end of the target RNA sequence, thereby giving the oligonucleotide more time to anneal before the RdRp processes through the region where the duplex between the labelled oligonucleotide and the target sequence should be formed.

The oligonucleotides, and also any ssRNA in the context of the present invention, may be prepared by any suitable methods. Methods for preparing oligonucleotides of specific sequence are known in the art, and include, for example, cloning and restriction of appropriate sequences and direct chemical synthesis. Chemical synthesis methods may include, for example, the phosphotriester method described by Narang et al. (1979) Method in Enzymology 68:90, the phoshpdiester method disclosed by Brown et al. (1979) Methods in Enzymology 68:109, the diethylphosphoramidate method disclosed in Beaucage et al. (1981) Tetrahedron Letters 22:1859, and the solid support method disclosed in US-A-4,458,066.

The oligonucleotide is labelled, as described below, by incorporating moieties detectable by spectroscopic, photochemical, biochemical, immunochemical, or chemical means. The method of linking or conjugating the label to the oligonucleotide depends, of course, on the type of label(s) used and the position of the label on the oligonucleotide.

A variety of labels that would be appropriate for use in the present invention, as well as methods for their inclusion in the oligonucleotide, are known in the art and include, but are not limited to, enzymes (for example alkaline phosphatase and horseradish peroxidase) and enzyme substrates, radioactive atoms, fluorescent dyes, chromophores, chemiluminescent labels, electrochemiluminescent labels, such as Origen™ (Igen), ligands having specific binding partners or any other labels that may interact with each other to enhance, alter, or diminish a signal.

Among radioactive atoms, ³²P is preferred. Methods for introducing ³²P into nucleic acids are known in the art, and include, for example 5'-labeling with a kinase, or random insertion by nick translation. Enzymes are typically detected by their activity. "Specific binding partner" refers to a protein capable of binding a ligand molecule with high specificity, as for example in the case of an antigen and a monoclonal antibody specific therefore. Other specific binding partners include biotin and avidin or streptavidin, IgG and protein A, and the numerous receptor-ligand couples known in the art. The above description is not meant to categorize the various labels into distinct classes, as the same label may serve in several different modes. For example¹²⁵I may serve as a radioactive label or as an electron-dense reagent. HRP may serve as enzyme or as antigen for a monclonal antibody. Further, one may combine various labels for a desired effect. For example, one might label a probe with biotin, and detect the presence of the oligonucleotide with avidin labelled with ¹²⁵I, or with an anti-biotin monoclonal antibody labelled with HRP. Other permutations and possibilities will be readily apparent for the skilled person and considered as equivalents within the scope of the present invention.

Fluorophores for use as labels in constructing the labelled oligonucleotide according to the present invention are preferred and include rhodamine and derivatives, such as Texas Red, 5-carboxytetramethyl rhodamine (5-TAMRA), 6-carboxytetramethyl rhodamine (6-TAMRA) and their respective succinimidyl esters, fluorescein and derivatives, such as 5-bromomethyl fluorescein, 5-carboxy fluorescein (5-FAM), 6-carboxy fluorescein (6-FAM), and their respective succinimidyl esters, Lucifer Yellow, IAEDANS, 7-Me₂-N-coumarin-4-acetate, 7-OH-4-CH₃-coumarin-3-acetate, 7-NH₂-4-CH₃-coumarin-3-acetate (AMCA), monobromobimane, pyrene trisulfonates, such as Cascade Blue, and monobromotrimethyl-ammoniobimane.

It is further preferred, that, if fluorescence is used to detect the released oligonucleotide, the ssRNA is provided with a molecule quenching the fluorescence of the fluorescent label of the oligonucleotide when it is hybridised to the target RNA. For example, the oligonucleotide may be labelled with a fluorescein derivative, such as 5- or 6-FAM, preferably at the 5'-end thereof, and the ssRNA is provided with a quencher for the fluorescein label, for example 5-TAMRA or 6-TAMRA.

Furthermore, in this situation it is also preferred that the oligonucleotide comprising the (fluorescein) label, preferably at its 5'-end, hybridises to the target sequence near its 5'-end which carries the quencher molecule. The quencher/donor may be generally selected as FRET (fluorescence resonance energy transfer) pairs such as in the case of FAM/TAMRA. However, it may also be desirable to select a so-called "dark quencher" (such as Dabcyl, methyl orange).

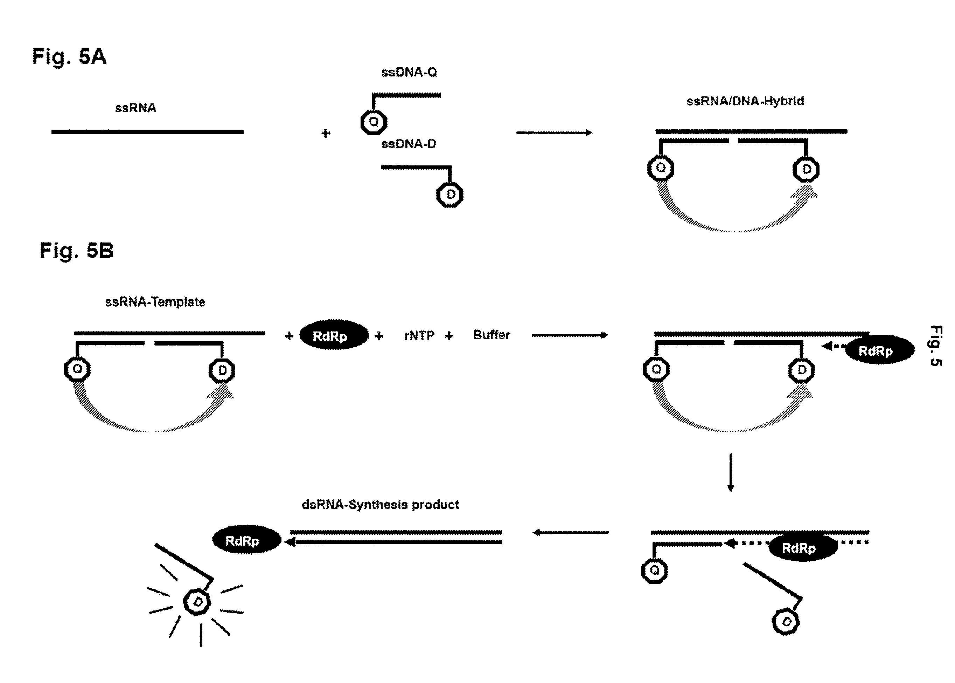

According to a further embodiment of the present invention the fluorescence donor-quencher pair can be provided in the form of two oligonucleotides (which may each be of RNA or DNA type) substantially complementary (with respect to the meaning of "substantially complementary" it is referred to the corresponding section above) to the target/template RNA but hybridising thereto at different, non-overlapping regions of said target/template RNA, and whereby the regions of complementarity to the target/template RNA are selected such that, when the oligonucleotides are hybridized to the target/template RNA, the fluorescence by the labelled oligonucleotide is quenched by the second oligonucleotide carrying the quencher, in particular by FRET between the fluorescence donor and quencher. Thus, when both donor and quencher oligonucleotides are hybridized to the target/template RNA, no or only a minor fluorescence signal is detected. When the RdRp according to the present invention initiates *de novo* RNA synthesis and synthesises an RNA strand complementary to the target/template RNA, it displaces both the donor and the quencher oligonucleotide from the target, and fluorescence emitted by the donor can be detected (see Fig. 5). In the illustrative example of Fig. 5, the quencher oligo has the quenching moiety at its 5'-end and hybridizes to or near the 5'-end of the target/template RNA. The donor oligo has the fluorescence donor at its 3'-end and hybridizes to or near the 3'end of the target/template RNA. Of course, quencher and donor moieties can be present at either probe. Furthermore, a quencher moiety could be present at the 3'-end of one oligo hybridizing to or near the 5'-end of the target/template and the donor group could be bound to the 5'-end of the other oligo (i.e. the labelled oligonucleotide) hybridizing near or to the 3'-end of the target/template RNA, and vice versa. In the latter scenario, it would be possible to use hybridizing regions which are farther remote from one another. With regard to preferred lengths and other characteristics of the quencher oliconucleotide (i.e. the second oligonucleotide present in step (a) or (i), respectively, of the above defined methods) it is referred to the above sections in connection with the labelled oligonucleotide (i.e. the donor oligo). Examples of useful donor/quencher pairs in the context of the present invention have already been described above. Particularly preferred examples for providing donor/quencher pairs in the form of two oligonucleotides are a 5- or 6-FAM labelled oligonucleotide (donor) and a 5- or 6-TAMRA-coupled oligonucleotide (quencher).

Further preferred embodiments of the methods according to the invention make use of so-called Molecular Beacons as labelled oligonucleotides. Molecular Beacons are described in US-A-5,925,517.

In this context, it should be noted that "hybridizing at the 5'-end" or hybridizing at the 3'-end" does necessarily mean that the labelled oligonucleotide (or second oligonucleotide having a quenching moiety) hybridises exactly to the 5'-end or 3'-end, respectively, of the target sequence. Rather, these terms also include that the oligonucleotide may hybridize near the 5'-end or 3'-end, respectively, which means that there may be additional nucleotides within the target sequence between its very 5'-end or 3'-end, respectively, of the sequence hybridizing with the respective oligonucleotide.

The labels for use in the present invention may be attached to the oligonucleotide directly or indirectly by a variety of techniques. Depending on the precise type of label used, the label can be located at the 5'- or the 3'-end of the oligonucleotide, located internally in the oligonucleotide or attached to spacer arms of various sizes and compositions to facilitate signal interactions. Using commercially available phosphoramidite reagents, one can produce oligomers containing functional groups (e.g., thiols or primary amines) at either the 5'- or the 3'-terminus via an appropriately protected phosphoramidite, and can label them using protocols described in, for example, PCR Protocols: A Guide to Methods and Applications (Innis et al., eds. Academic Press, 1990). The same holds true for chemical quenching moieties for an optionally present quencher oligonucleotide.

Methods for introducing oligonucleotide functionalizing reagents to introduce one or more sulfhydryl, amino or hydroxyl moieties into the oligonucleotide, typically at the 5'-terminus, are described in US-A4,914,210. A 5'-phosphate group can be introduced as a radioisotope by using polynucleotide kinase and gamma-³²P-ATP to provide a reporter group. Biotin can be added to the 5'-end by reacting an aminothymidine residue, or a 6-amino hexyl residue, introduced during synthesis, with an N-hydroxysuccinimide ester of biotin. Labels at the 3'-terminus may employ polynucleotide terminal transferase to add the desired moiety, such as for example, cordycepin³⁵S-dATP, and biotinylated dUTP.

Oligonucleotide derivatives are also available labels. For example, etheno-dA and etheno-A are known fluorescent adenine nucleotides that can be incorporated into an oligonucleotide. Similarly, etheno-dC or 2-amino purine deoxyriboside is another analogue that could be used in oligonucleotide synthesis. The oligonucleotides containing such nucleotide derivatives may be hydrolyzed to release much more strongly fluorescent mononucleotides as the RdRp unwinds the duplex formed between the target ssRNA and the labelled oligonucleotide.

According to the present invention, the term "RNA polymerization conditions" means the conditions, in particular relating to buffer, temperature, salt and metal ion (if applicable), that allow the RdRp to synthesize an RNA strand complementary to a template strand in the absence of a primer. Appropriate buffer, salt, metal ion, reducing agent (if applicable) and other conditions of RdRps are known to the skilled person. With regard to RdRps of caliciviruses, it is referred to WO-A-2007/012329. Thus, in typical examples of the methods according to the present invention, the ssRNA template is used in amounts of e.g. 1 µg to 4 µg per 50 µl reaction volume. The concentration of the ribonucleoside triphosphates is preferably in the range of from 0.1 µmol/l to 1 µmol/l, for example 0.4 µmol/l. The concentration of the RdRp may be for example 1 µmol/l to 6 µmol/l.

Typical buffer conditions are 10 to 80 mM, more preferred 20 to 50 mM HEPES pH 8.0, 1 to 4 mM, for example 3 mM magnesium acetate, magnesium chloride, manganese acetate or manganese chloride and 1 to 4 mM of a reducing agent, for example DTT. With respect to the terminal transferase activity of the RdRp, substantially the same conditions apply except that only one ribonucleotide is present, e.g. rCTP for providing a C-terminal repeat.

The method according to the present invention may be stopped by introducing a stop solution into the reaction mixture. A typical stop solution contains 2 to 10 mM, preferably 4 to 8 mM ammonium acetate and 50 to 200 mM, for example 100 mM EDTA.

The products of the method according to the invention are duplex molecules consisting of the template strands and the complementary strands synthesized de *novo* by the RdRp. By-products of this synthesis are the displaced labelled oligonucleotides. As contemplated in the RNA amplification a protocol according to the present invention, the duplexes formed from the ssRNA and the complementary strand polymerized by the RdRp may be separated and the steps of annealing the labelled oligonucleotide (and, optionally, a second oligonucleotide having a quencher moiety), polymerization of complementary strands and separating the RNA duplexes may be repeated, for example 2 to 50 or up to 200, more preferred 15 to 40, most preferred 20 to 30 times.

The strand separation may be carried out by application of heat (heat denaturation), by chemical denaturation or enzymatically, preferably by a helicase. Especially in the case of heat denaturation it is desirable to add further RdRp molecules to the reaction mixture before performing the next polymerization step.

For practicing the methods of the present invention, a thermal cycler, such as the commercially available machines from Perkin-Elmer Instruments or Roche Diagnostics may be employed.

The detection or verification of the labelled oligonucleotide displaced by the action of the RdRp may be accomplished by a variety of methods and may be dependent on the source of the label or labels employed. One convenient embodiment of the invention is to subject the reaction products, including the released labelled oligonucleotide, to size analysis. Methods for determining the size of the labelled nucleic acid fragments are known in the art, and include, for example, gel electrophoresis, sedimentation ingredients, gel exclusion chromatography and homochromatography.

The reagents applied in the methods according to the present invention can be packaged into diagnostic ktis. Diagnostic kits include the labelled oligonucleotide and the RdRp. The labelled oligonucleotide is preferably blocked at its 3'-end and comprises a label as described above. The kit may further contain a second oligonucleotide as described above comprising a quenching moiety, preferably at its 5'- or 3'-end. If the quencher moiety is present at the 5'-end, the second oligonucleotide is preferably blocked at its 3'-end. The kit may also contain other suitably packaged reagents and materials needed for carrying out the methods according to the present invention, for example, buffers, ribonucleotides (rATP, rGTP, rCTP, rUTP, and, optionally, a stop solution (preferably a stop solution as defined above, more preferred in the form of a 5x or 10x stop solution) and, also optionally, a helicase as well as instructions for conducting the methods.

The figures show:

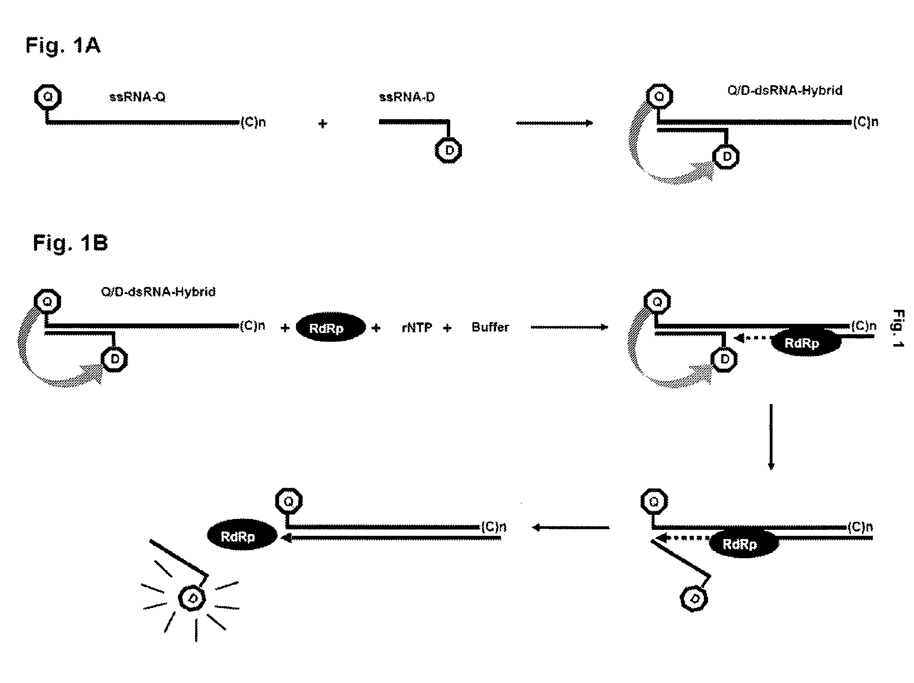

- Fig. 1: is a schematic illustration of a preferred embodiment of the RNA detection method according to the present invention showing the generation of a quencher/donor double-stranded RNA hybrid (Q/D-dsRNA-hybrid) used as a template. Fig. 1A: The hybrid is generated by hybridizing the single-stranded RNA olgionucleotide labelled at its 5'-terminus with the quencher (ssRNA-Q) and the single-stranded RNA labelled at its 5'-terminus with the donor (ssRNA-D). The ssRNA-Q and ssRNA-D bear complementary sequences. Emission of energy by the donor is attenuated by the quencher through resonance energy transfer. The hybridization reaction mixture contains equimolar concentrations of ssRNA-Q and ssRNA-D, incubated in the hybridization buffer at 65 °C for 30 min, then chilled on ice for 15 min. The hybridization buffer contains Tris-HCl 10 mM pH 8.0, NaCl 20 mM, EDTA 1 mM. Fig. 1B: schematic representation of strand displacement by the calicivirus RNA-dependent RNA polymerase (RdRp). The RdRp synthesizes RNA starting at the 3'-terminus of the template (ssRNA-Q), and displacing the ssRNA-D hybridized at the 5'-end of the ssRNA-Q. The ssRNA-Q template strand has a (C)ₙ nucleotide sequence at its 3'-terminus (with n ≥ 3). RNA synthesis results in a double-stranded RNA molecule consisting of the single-stranded RNA labelled at its 5'-terminus with the quencher (ssRNA-Q) and its complementary single-stranded RNA synthesized by the RdRp, as indicated. Strand displacement leads to release of the ssRNA-D from the ssRNA-Q, with subsequent detection of the energy emitted by the donor.

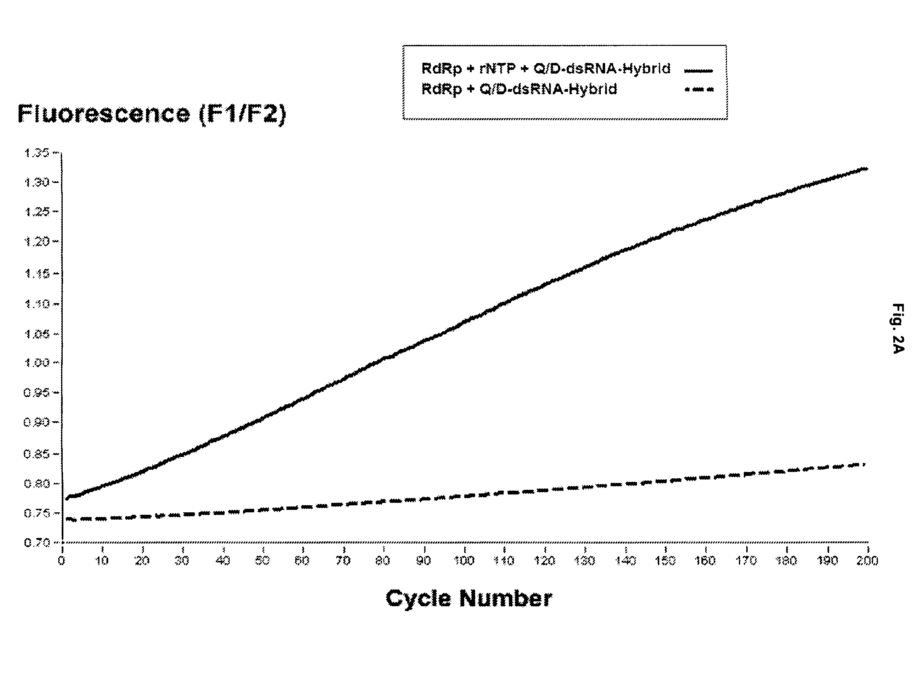

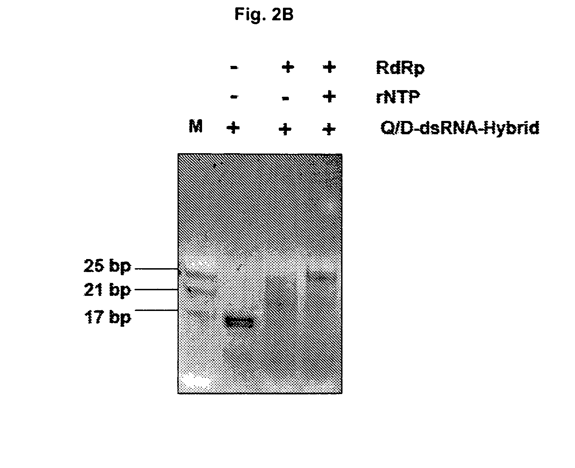

- Fig. 2: shows results of experiments demonstrating strand displacement of a labelled oligoribonucleotide from a template ssRNA strand by calicivirus RdRp. Fig. 2A: graphical representation of fluoresecence emission dependent on reaction cycle number. Filled line: reaction containing calicivirus RdRp, ribonucleotide triphosphates (rNTP), RdRp buffer, and Q/D-dsRNA hybrid. Dashed line: negative control reaction in the absence of rNTPs. Fig. 2B: photopgraph showing the products of the reactions of Fig. 2A analyzed using native polyacrylamide gel electrophoresis (PAGE). Lanes (from left to right): M: molecular size marker (base pairs (bp) are indicated); 1^{st} reaction lane: reaction containing Q/D-dsRNA hybrid only; 2^{nd} reaction lane: control reaction containing RdRp and Q/D-dsRNA hybrid, but no rNTPs; 3^{rd} reaction lane: reaction containing all required components (RdRp, rNTPs, Q/D-dsRNA hybrid).

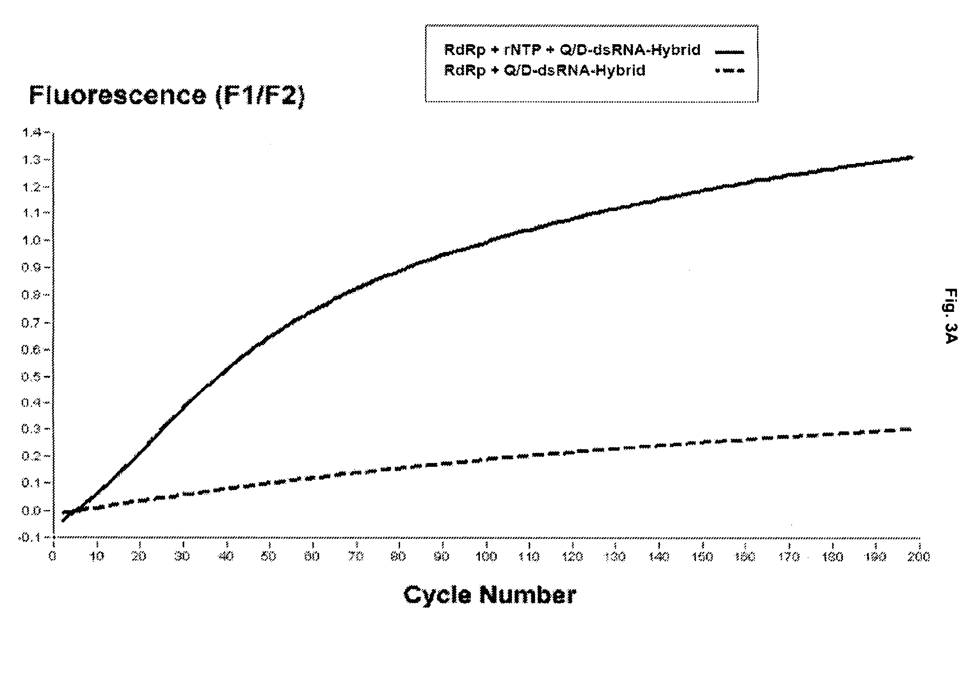

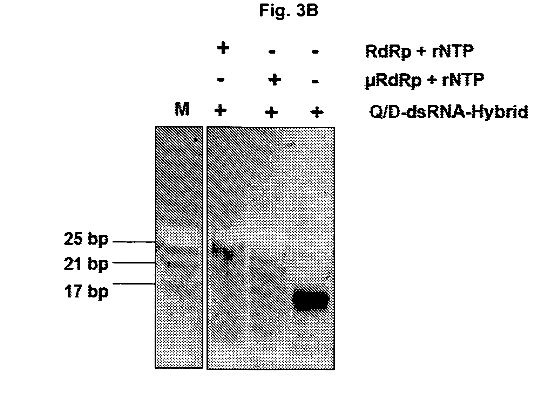

- Fig. 3: shows results of further experiments demonstrating strand displacement of a labelled oligoribonucleotide from a template ssRNA strand by calicivirus RdRp. Fig. 3A: graphical representation of fluorescence emission dependent on reaction cycle number. Filled line: reaction containing calicivirus RdRp, ribonucleotide triphosphates (rNTP), RdRp buffer, and Q/D-dsRNA hybrid. Dashed line: control reaction in the presence of an active site mutant of the RdRp (µRdRp) leading to total inhibition of RNA synthesis by the enzyme. Fig. 3B: photograph showing the products of the reactions of Fig. 3A analyzed using native polyacrylamide gel electrophoresis (PAGE). Lanes (from left to right): M: molecular size marker (base pairs (bp) are indicated); 1^{st} reaction lane: reaction containing calicivirus RdRp, rNTPs and Q/D-dsRNA hybrid; 2^{nd} reaction lane: control reaction containing µRdRp, rNTPs and Q/D-dsRNA hybrid; 3^{rd} reaction lane: control reaction containing Q/D-dsRNA hybrid only.

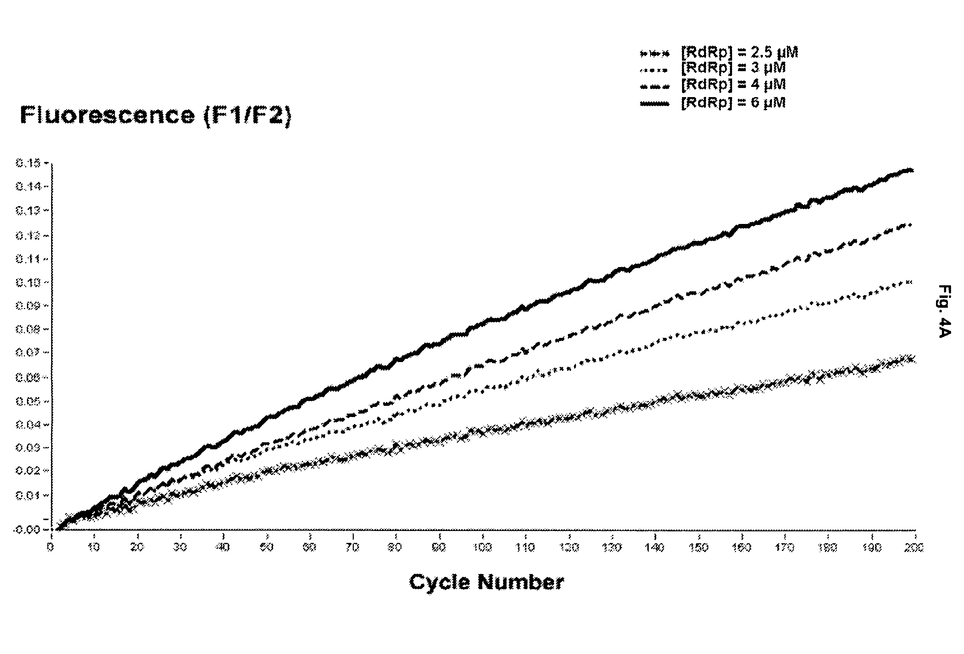

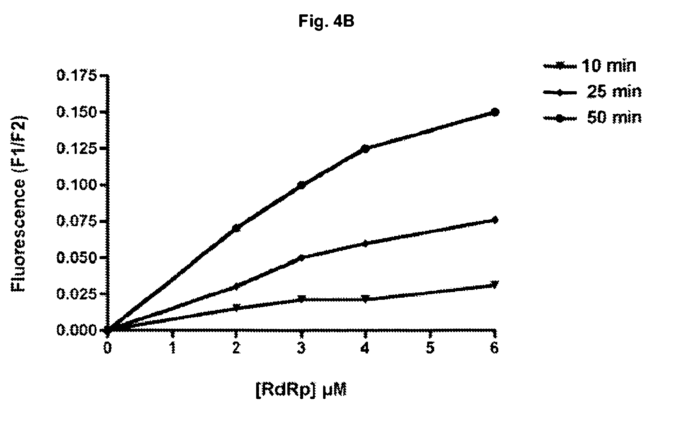

- Fig. 4: shows the results of experiments demonstrating the concentration- and time-dependent synthesis of dsRNA through strand displacement by the calicivirus RdRp. Fig. 4A: graphical representation of fluoresecence emission dependent on reaction cycle number. The reaction mix contained increasing concentrations of RdRp (2.5 µM, 3 µM, 4 µM, and 6 µM; as indicated). Fig. 4B: graphical representation of fluorescence signal depending on the concentration of the calicivirus RdRp and shown for different reaction times as indicated.
- Fig. 5: shows schematic representations of a further embodiment of the RNA detection method according to the present invention illustrating a fluorescence resonance energy transfer assay (FRET) using the calicivirus RNA-dependent RNA polymerase (RdRp) and a single stranded RNA as the template in the presence a labeled DNA oligoprobes having a fluorescence donor (D) and a second oligoprobe coupled to a fluorescence quencher (Q). Fig. 5A: an ssRNA-DNA hybrid is generated by hybridizing the single-stranded RNA to a labeled DNA oligoprobe having a fluorescence donor at its 5'-end and a second DNA oligoprobe containing a fluorescence quencher at its 3'-end. The oligoprobe containing a donor at its 5'-end is complementary to the 3'-terminus of the template RNA, and the oligoprobe containing the quencher is complementary to the 5'-terminus of the template RNA. Emission of energy by the donor is attenuated by the quencher through resonance energy transfer. Fig. 5B: schematic representation of strand displacement by the calicivirus RNA-dependent RNA polymerase (RdRp). The RdRp synthesizes RNA starting at the 3'-terminus of the template (ssRNA-Template), and displacing the oligoprobe-DNA-donor hybridized at the 3'-end of the ssRNA-template. RNA synthesis results in a double stranded RNA molecule. Strand displacement leads to release of the oligoprobe-DNA-donor and the Oligoprobe-DNA-quencher from the ssRNA-template, with subsequent detection of the fluorescence emitted by the donor.

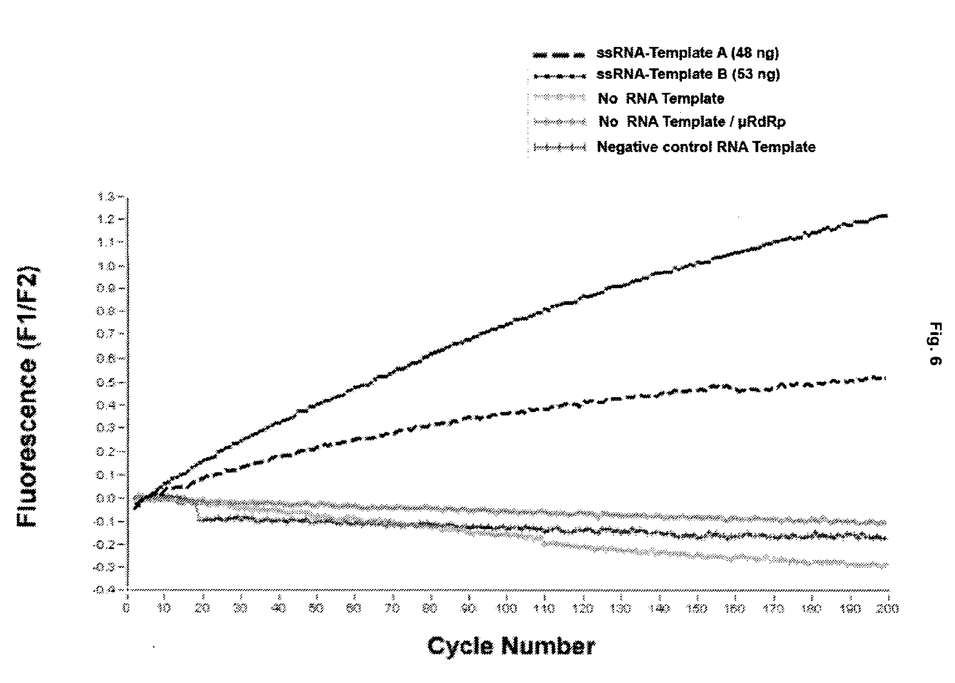

- Fig. 6: shows a graphical representation of fluorescence emission dependent on the number of reaction cycles which illustrates an example of fluorescence resonance energy transfer assay (FRET) using the calicivirus RNA-dependent RNA polymerase (RdRp) and a single stranded RNA as the template in the presence of a labeled DNA oligoprobe containing a fluorescence donor and a second oligoprobe having a fluorescence. In this experiment, two different ssRNA templates (template A and template B) in different amounts (ng as indicated) were used. Template B is miR-375 and has the sequence (5'-UUUGUUCGUUCGGCUCGCGUGA-3', SEQ ID NO: 9). Template A is modified form of miR-375 having the sequence 5'-UUUGUUCGUUCGGCUCGCGUGACCC-3' (SEQ ID NO: 10). µRdRp: active site mutant of calicivirus RdRp. Negative control RNA template: ssRNA having no complementarity to the oligoprobes used.

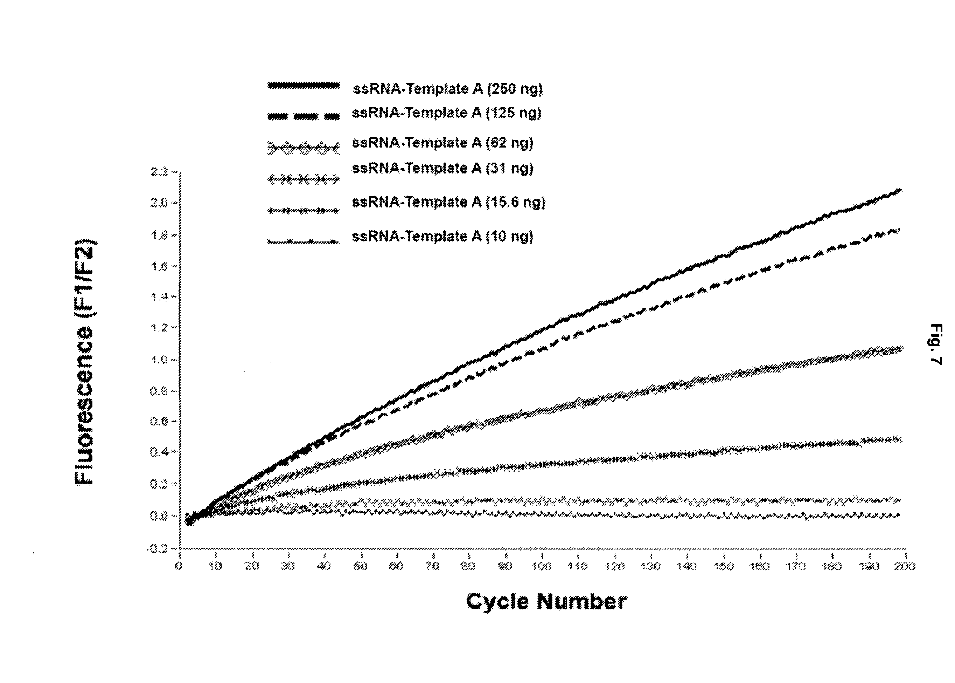

- Fig. 7: shows a graphical representation of fluorescence emission dependent on the number of reaction cycles which illustrates the dependency of the synthesis of dsRNA through strand displacement by the calicivirus RdRp on the template concentration. Increasing amounts of template A (from 10 to 250 ng, as indicated) were used. Template A is the same as described in Fig. 6.

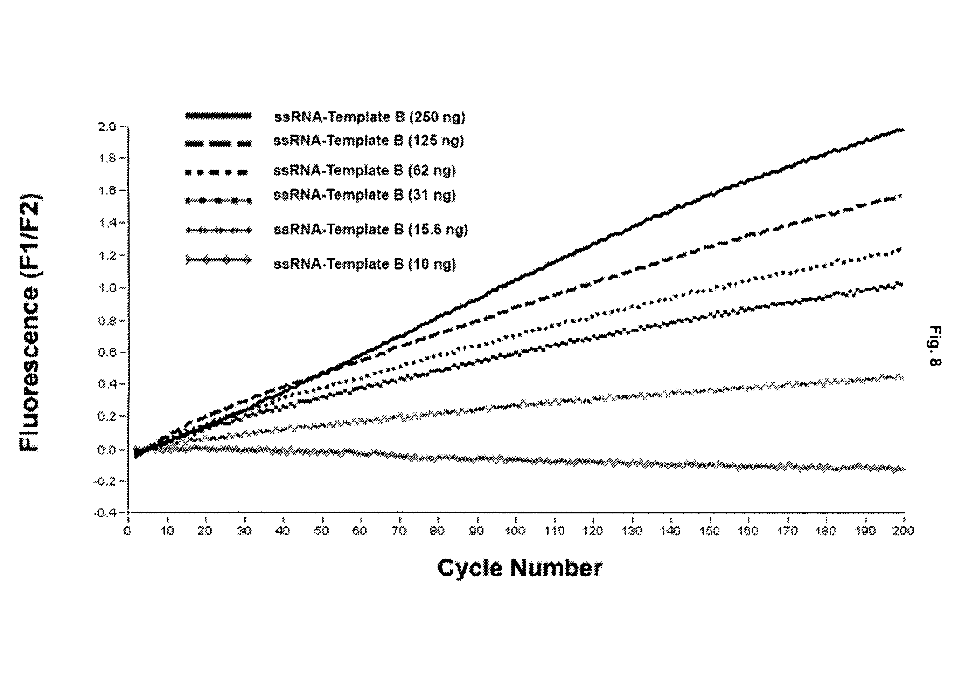

- Fig. 8: shows a further graphical representation of fluorescence emission dependent on the number of reaction cycles which illustrates the dependency of the synthesis of dsRNA through strand displacement by the calicivirus RdRp on the template concentration. Increasing amounts of template B (from 10 to 250 ng, as indicated) were used. Template B is the same as described in Fig. 6.

The present invention is further illustrated by the following non-limiting examples.

### EXAMPLES

### Example 1: Strand displacement assay using calicivirus RdRp

The reaction mix (25 µl) contains the RdRp (sapovirus; 6 µM), the Q/D-dsRNA-hybrid at a final concentration of 4 µM, the RdRp-Buffer (consisting of HEPES pH 8.0 4 mM, MnCl₂ 0.6 mM, ammonium acetate 25 mM, DTT 0.25 mM), ATP, UTP, CTP, GTP (designed as ribonucleotides triphosphates, rNTP) at a final concentration of 0.4 mM each. The quencher used is TAMRA, and the donor is 6-FAM. Fluorescence is monitored in real-time on the LightCycler 1.5 machine (Roche, Basel, Switzerland). Each cycle corresponds to a 15 seconds incubation of the reaction at 30°C, with subsequent measurement of fluorescence emission. The increase of the fluorescence over a period of 3000 seconds (200 cycles x 15 seconds = 3000 seconds) is shown in Fig. 2A. The increase of fluorescence Fig. 2A, (filled line) was observed in a reaction consisting of the Calicivirus RdRp, the ribonucleotide triphosphates (rNTP), the RdRp buffer, and the Q/D-dsRNA hybrid as mentioned previously. As a control, the same reaction was run in the absence of rNTP (Fig. 2A, dashed line).

The products of the reactions were analyzed using native polyacrylamide gel electrophoresis (PAGE). The RdRp synthesizes a double-stranded RNA product only in the presence of all components of the reaction as mentioned above. Synthesis of the dsRNA occurs by strand displacement, leading to a double-stranded RNA molecule consisting of the single-stranded RNA labelled at its 5'-terminus with the quencher (ssRNA-Q) and its complementary single-stranded RNA synthesized by the RdRp, as shown in Fig. 2B. When rNTPs are omitted, no dsRNA is synthesized, as shown Fig. 2B as well. The reaction educt used in both reactions is denoted in Fig. 2B as "Q/D-dsRNA-Hybrid".

### Example 2: Strand displacement does not occur with active-site mutant of calicivirus RdRp

The same reactions were carried out as in Example 1, but using an active site mutant of the sapovirus RdRp (µRdRp) in a control reaction. The results are shown in Fig. 3.

Thus, the reaction mix (25 µl) contains the RdRp (6 µM), the Q/D-dsRNA-Hybrid at a final concentration of 4 µM, the RdRp buffer (consisting of HEPES pH 8.0 4 mM, MnCl₂ 0.6 mM, ammonium acetate 25 mM, DTT 0.25 mM), ATP, UTP, CTP, GTP (here designed as ribonucleotides triphosphates, rNTP) at a final concentration of 0.4 mM each. The quencher used is TAMRA, and the donor is 6-FAM. Fluorescence is monitored in real-time on the LightCycler 1.5 machine (Roche, Basel, Switzerland). Each cycle corresponds to a 15 seconds incubation of the reaction at 30°C, with subsequent measurement of fluorescence emission. The increase of the fluorescence over a period of 3000 seconds (200 cycles x 15 seconds = 3000 seconds) is monitored. The increase of fluorescence (filled line) was observed in a reaction containing the sapovirus RdRp, the ribonucleotide triphosphates (rNTP), the RdRp-Buffer, and the Q/D-dsRNA hybrid as mentioned previously. As a control, the same reaction was run in the presence of an active site mutant of the RdRp (µRdRp) leading to total inhibition of RNA synthesis by the enzyme (dashed line in Fig. 3A).

The products of the reactions were analyzed using native polyacrylamide gel electrophoresis (PAGE). Synthesis of the dsRNA occurs by strand displacement, leading to a double-stranded RNA molecule consisting of the single-stranded RNA labelled at its 5'-terminus with the quencher (ssRNA-Q) and its complementary single-stranded RNA synthesized by the sapovirus RdRp, as indicated in Fig. 3B. When the µRdRp is used, no dsRNA is synthesized, as shown in Fig. 3B. The reaction educt used in both reactions is the Q/D-dsRNA hybrid.

### Example 3: Concentration and time dependency of dsRNA synthesis through strand displacement by calicivirus RdRp.

The reaction mix contained increasing concentrations of sapovirus RdRp (2.5 µM, 3 µM, 4 µM, and 6 µM; as indicated), the Q/D-dsRNA-hybrid at a final concentration of 4 µM, the RdRp buffer (HEPES pH 8.0 4 mM, MnCl₂ 0.6 mM, ammonium acetate 25 mM, DTT 0.25 mM), ATP, UTP, CTP, GTP (here designated as ribonucleotides triphosphates, rNTP) at a final concentration of 0.4 mM each. The quencher used is TAMRA, and the donor is 6-FAM. Fluorescence is monitored in real-time on the LightCycler 1.5 machine (Roche, Basel, Switzerland). Each cycle corresponds to a 15 seconds incubation of the reaction at 30°C, with subsequent measurement of fluorescence emission. The increase of the fluorescence over a period of 3000 seconds (200 cycles x 15 seconds = 3000 seconds) is shown in Fig. 4A for all four different concentrations of the calicivirus RdRp.

For elucidating the time-dependency of RNA synthesis by the calicivirus RdRp through strand-displacement, the reaction mix (25 µl) contained increasing concentrations of sapovirus RdRp (2.5 µM, 3 µM, 4 µM, and 6 µM), the Q/D-dsRNA-hybrid at a final concentration of 4 µM, the RdRp-Buffer (consisting of HEPES pH 8.0 4 mM, MnCl₂ 0.6 mM, ammonium acetate 25 mM, DTT 0.25 mM), ATP, UTP, CTP, GTP (here designed as ribonucleotides triphosphates, rNTP) at a final concentration of 1 mM each. The quencher used is TAMRA, and the donor is 6-FAM. Fluorescence is monitored in real-time on the LightCycler 1.5 machine at 10 min, 25 in and 50 min, as indicated in Fig. 4B. Fig. 4B demonstrates that the increase of the fluorescence is dependent of the RdRp concentration and the reaction time.

### Example 4: RNA strand displacement assay using two oligonucleotide probes

The reaction mix (25 µl) contains the RdRp (sapovirus; 7.5 µM), a quencher-oligoprobe (Q) and a donor-oligoprobe (D) at a final concentration of 0.5 µM, the RdRp buffer (containing HEPES pH 8.0 4 mM, MnCl₂ 0.6 mM, ammonium acetate 25 mM, DTT 0.25 mM), ATP, UTP, CTP (designated as ribonucleotides triphosphates, rNTP) at a final concentration of 0.4 mM each and GTP at a concentration of 2 mM. The fluorescence quencher used is TAMRA, and the fluorescence donor is 6-FAM. Fluorescence is monitored in real-time on the LightCycler 1.5 machine (Roche, Basel, Switzerland). Each cycle corresponds to a 15 seconds incubation of the reaction at 30°C, with a subsequent measurement of fluorescence emission. The increase of the fluorescence over a period of 3000 seconds (200 cycles x 15 seconds = 3000 seconds) is measured. The results of the experiments are shown in Fig. 6.

An increase of fluorescence was observed in a reaction containing the calicivirus RdRp, the ribonucleotide triphosphates (rNTPs), RdRp-buffer, the quencher-oligoprobe (Q), the donor-oligoprobe (D) and either 53 ng of template B (miR-375: 5'-UUUGUUCGUUCGGCUCGCGUGA-3', SEQ ID NO: 8) or 48 ng of template A (modified sequence of miR-375: 5'-UUUGUUCGUUCGGCUCGCGUGACCC3', SEQ ID NO: 9). As a control, the same reaction was run in the presence of an active site mutant of the RdRp (µRdRp) leading to total inhibition of RNA synthesis by the enzyme (µRdRp), and subsequent absence of fluorescence signal. When no RNA template is present in the reaction, or a negative control ssRNA-template not complementary to the quencher-oligoprobe (Q) and the donor-oligoprobe (D) is used, no signal is detected.

### Example 5: Dependency of strand displacement assay on template concentration

The reaction mix (25 µl) contains the RdRp (sapovirus; 7.5 µM), the quencher-oligoprobe (Q) and the donor-oligoprobe (D) at a final concentration of 0.5 µM, the RdRp-Buffer (consisting of HEPES pH 8.04 mM, MnCl₂ 0.6 mM, ammonium acetate 25 mM, DTT 0.25 mM), ATP, UTP, CTP (here designated as ribonucleotides triphosphates, rNTP) at a final concentration of 0.4 mM each and GTP at concentration of 2 mM. The quencher used is TAMRA, and the donor is 6-FAM. Fluorescence is monitored in real-time on the LightCycler 1.5 machine (Roche, Basel, Switzerland). The reactions contain different concentrations of template A or template B (10 ng, 15.6 ng, 31 ng, 62 ng, 125 ng, 250 ng). Each cycle corresponds to a 15 seconds incubation of the reaction mixture at 30°C, with subsequent measurement of fluorescence emission. The increase of the fluorescence over a period of 3000 seconds (200 cycles x 15 seconds = 3000 seconds) is measured. The increase of fluorescence was observed in a reaction containing the calicivirus RdRp, the ribonucleotide triphosphates (rNTP), RdRp-buffer, and the quencher-oligoprobe (Q) and the donor-oligoprobe (D).

As shown in Fig. 7 and Fig. 8, a detectable increase in fluorescence emission is observed at template concentrations above 10 ng (template B) or 15.6 ng (template A), respectively.

## Claims

1. A method for the detection of a target RNA sequence in a sample comprising the steps of:
(a) contacting single-stranded RNA (ssRNA) molecules present in a sample with a labelled oligonucleotide containing a sequence substantially complementary to a region of the target RNA under hybridisation conditions to provide a mixture of RNA-oligonucleotide duplexes wherein the RNA-oligonucleotide duplexes comprise the target RNA annealed to the labelled oligonucleotide;
(b) maintaining the mixture of step (a) with an RNA-dependent RNA polymerase (RdRp) under RNA polymerisation conditions in the absence of a primer wherein said RdRp has an RNA-oligonucleotide duplex separation activity and is capable of synthesising a complementary RNA strand on a single-stranded RNA template without requiring an RNA duplex for initiation of polymerisation such that the RdRp polymerises an RNA strand complementary to the ssRNA in the sample and releases the labelled oligonucleotide; and
(c) detecting and/or measuring the signal generated by the released labelled oligonucleotide.

2. An RNA amplification method comprising the steps of:
(i) contacting a single-stranded RNA (ssRNA) template with a labelled oligonucleotide containing a sequence substantially complementary to a region of said ssRNA under hybridisation conditions to provide RNA-oligonucleotide duplexes comprising the ssRNA annealed to the labelled oligonucleotide;
(ii) maintaining the RNA-oligonucleotide duplexes of step (i) with an RNA-dependent RNA polymerase (RdRp) under RNA polymerisation conditions in the absence of a primer wherein said RdRp has an RNA-oligonucleotide duplex separation activity and is capable of synthesising a complementary RNA strand on a single-stranded RNA template without requiring an RNA duplex for initiation of polymerisation such that the RdRp polymerises an RNA strand complementary to the ssRNA and releases the labelled oligonucleotide;
(iii) separating the RNA duplexes formed by the RdRp between the ssRNA and the polymerised complementary strand;
(iv) optionally, repeating steps (i) to (iii); and
(v) detecting and/or measuring the signal generated by the released labelled oligonucleotide.

3. The method of claim 1 or 2 wherein the RdRp is an RdRp of a virus of the *Caliciviridae* family.

4. The method according to any one of the preceding claims wherein the ssRNA in the sample or the ssRNA template, respectively, has at least one C at its 3'-end.

5. The method of claim 4 wherein the ssRNA in the sample or the ssRNA template, respectively, has a (C)ₙ repeat at its 3'-end, with n ≥ 3.

6. The method according to any one of the preceding claims wherein the labelled oligonucleotide contains a fluorescent label.

7. The method of claim 6 wherein the oligonucleotide contains a fluorescent label at its 5'-end.

8. The method of claim 6 or 7 wherein the ssRNA is provided with a molecule quenching the fluorescence of the fluorescent label of the oligonucleotide when hybridised to the target RNA.

9. The method of claim 8 wherein the labelled oligonucleotide hybridises to the 5'-end of the target RNA.

10. The method of claim 6 wherein a second oligonucleotide is hybridised in step (a) or (i), respectively, to the ssRNA which second oligonucleotide is substantially complementary to a region of the ssRNA which does not overlap with the region of complementarity of the labelled oligonucleotide and wherein the second oligonucleotide contains a chemical moiety capable of quenching the fluorescence of the fluorescent label of the labelled oligonucleotide when both the labelled and the second oligonucleotide are hybridised to the ssRNA.

11. The method according to any one of the preceding claims wherein the labelled oligonucleotide and, optionally, the second oligonucleotide has/have a length of from 5 to 20, preferably 10 to 12, nucleotides.

12. The method according to any one of the preceding claims wherein the ssRNA has a length of from 16 to 40, preferably 18 to 25, nucleotides.

13. The method according to any one of the preceding claims wherein the ssRNA is microRNA or disrupted microRNA.

## Patentansprüche

1. Verfahren zur Detektion einer Ziel-RNA-Sequenz in einer Probe, umfassend die Schritte:
(a) Inkontaktbringen von in einer Probe vorliegenden einzelsträngigen RNA-(ssRNA) Molekülen unter Hybridisierungsbedingungen mit einem markierten Oligonukleotid, das eine Sequenz enthält, die zu einem Bereich der Ziel-RNA komplementär ist, um ein Gemisch von RNA-Oligonukleotid-Doppelsträngen bereitzustellen, wobei die RNA-Oligonukleotid-Doppelstränge die mit dem markierten Oligonukleotid hybridisierte Ziel-RNA umfassen;
(b) Inkubieren des Gemischs von Schritt (a) mit einer RNA-abhängigen RNA-Polymerase (RdRp) unter RNA-Polymerisationsbedingungen in Abwesenheit eines Primers, wobei die RdRp eine RNA-Oligonukleotid-Doppelstrangtrennungsaktivität aufweist und zur Synthese eines komplementären RNA-Strangs auf einer einzelsträngigen RNA-Matrize befähigt ist, ohne einen RNA-Doppelstrang zur Polymerisationsinitiation zu benötigen, so dass die RdRp einen zu der ssRNA in der Probe komplementären RNA-Strang polymerisiert und das markierte Oligonukleotid freisetzt; und
(c) Detektieren und/oder Messen des durch das freigesetzte markierte Oligonukleotid erzeugten Signals.

2. RNA-Amplifikationsverfahren, umfassend die Schritte:
(i) Inkontaktbringen von in einer Probe vorliegenden einzelsträngigen RNA-(ssRNA) Molekülen unter Hybridisierungsbedingungen mit einem markierten Oligonukleotid, das eine Sequenz enthält, die zu einem Bereich der ssRNA komplementäre Sequenz enthält, um ein Gemisch von RNA-Oligonukleotid-Doppelsträngen bereitzustellen, welche die mit dem markierten Oligonukleotid hybridisierte ssRNA umfassen;
(ii) Inkubieren des Gemischs von Schritt (i) mit einer RNA-abhängigen RNA-Polymerase (RdRp) unter RNA-Polymerisationsbedingungen in Abwesenheit eines Primers, wobei die RdRp eine RNA-Oligonukleotid-Doppelstrangtrennungsaktivität aufweist und zur Synthese eines komplementären RNA-Strangs auf einer einzelsträngigen RNA-Matrize befähigt ist, ohne einen RNA-Doppelstrang zur Polymerisationsinitiation zu benötigen, so dass die RdRp einen zu der ssRNA in der Probe komplementären RNA-Strang polymerisiert und das markierte Oligonukleotid freisetzt;
(iii) Trennen der von der RdRp zwischen der ssRNA und dem polymerisierten komplementären Strang gebildeten RNA-Doppelstränge;
(iv) ggf. Wiederholen der Schritte (i) bis (iii); und
(v) Detektieren und/oder Messen des durch das freigesetzte markierte Oligonukleotid erzeugten Signals.

3. Verfahren nach Anspruch 1 oder 2, wobei die RdRp eine RdRp eines Virus der Familie der *Caliciviridae* ist.

4. Verfahren nach einem der vorhergehenden Ansprüche, wobei die ssRNA in der Probe bzw. die ssRNA-Matrize mindestens ein C am 3'-Ende aufweist.

5. Verfahren nach Anspruch 4, wobei die ssRNA in der Probe bzw. die ssRNA-Matrize eine (C)ₙ-Wiederholung am 3'-Ende mit n ≥ 3 aufweist.

6. Verfahren nach einem der vorhergehenden Ansprüche, wobei das markierte Oligonukleotid eine Fluoreszenzmarkierung enthält.

7. Verfahren nach Anspruch 6, wobei das Oligonukleotid eine Fluoreszenzmarkierung am 5'-Ende aufweist.

8. Verfahren nach Anspruch 6 oder 7, wobei die ssRNA ein Molekül aufweist, das die Fluoreszenz der Fluoreszenzmarkierung des Oligonukleotids quencht, wenn dieses mit der Ziel-RNA hybridisiert ist.

9. Verfahren nach Anspruch 8, wobei das markierte Oligonukleotid mit dem 5'-Ende der Ziel-RNA hybridisiert.

10. Verfahren nach Anspruch 6, wobei im Schritt (a) bzw. (i) ein zweites Oligonukleotid mit der ssRNA hybridisiert wird, wobei das zweite Oligonukleotid im Wesentlichen komplementär zu einem Bereich der ssRNA ist, der nicht mit dem Bereich der Komplementarität des markierten Oligonukleotids überlappt und wobei das zweite Oligonukleotid eine chemische Einheit enthält, die zum Quenchen der Fluoreszenz der Fluoreszenzmarkierung des markierten Oligonukleotids befähigt ist, wenn sowohl das markierte als auch das zweite Oligonukleotid mit der ssRNA hybridisiert sind.

11. Verfahren nach einem der vorhergehenden Ansprüche, wobei das markierte Oligonukleotid und ggf. das zweite Oligonukleotid eine Länge von 5 bis 20, vorzugsweise 10 bis 12 Nukleotide aufweist/aufweisen.

12. Verfahren nach einem der vorhergehenden Ansprüche, wobei die ssRNA eine Länge von 16 bis 40, vorzugsweise 18 bis 25 Nukleotide aufweist.

13. Verfahren nach einem der vorhergehenden Ansprüche, wobei die ssRNA microRNA oder deregulierte microRNA ist.

## Revendications

1. Méthode de détection d'une séquence d'ARN cible dans un échantillon, comprenant les étapes :
(a) de mise en contact de molécules d'ARN simple brin (ssARN) présentes dans un échantillon avec un oligonucléotide marqué contenant une séquence sensiblement complémentaire à une région de la séquence d'ARN cible dans des conditions d'hybridation permettant d'obtenir un mélange de duplex ARN-oligonucléotides dans lequel les duplex ARN-oligonucléotides contiennent l'ARN cible annellé à l'oligonucléotide marqué,
(b) de maintien du mélange obtenu à l'étape (a) avec une ARN polymérase ARN-dépendante (RdRp) dans des conditions de polymérisation de l'ARN en absence d'une amorce dans laquelle ladite RdRp a une activité séparatrice du duplex ARN-oligonucléotide et est capable de synthétiser le brin complémentaire d'ARN sur un modèle simple brin sans nécessiter un duplex ARN pour initier la polymérisation, de sorte que la RdRp polymérise un brin d'ARN complémentaire au ssARN dans l'échantillon et relargue l'oligonucléotide marqué, et
(c) de détection et de mesure du signal généré par le relargage de l'oligonucléotide marqué.

2. Méthode d'amplification d'ARN comprenant les étapes de :
(i) de mise en contact d'un modèle d'ARN simple brin (ssARN) avec un oligonucléotide marqué une séquence sensiblement complémentaire à une région dudit ssARN dans des conditions d'hybridation permettant d'obtenir un mélange de duplex ARN-oligonucléotides comprenant le ssARN annellé à l'oligonucléotide marqué,
(ii) de maintien des duplex ARN-oligonucléotides de l'étape (i) avec une ARN polymérase ARN-dépendante (RdRp) dans des conditions de polymérisation de l'ARN en absence d'une amorce dans laquelle ladite RdRp a une activité séparatrice du duplex ARN-oligonucléotide et est capable de synthétiser le brin complémentaire d'ARN sur un modèle simple brin sana nécessiter un duplex ARN pour initier la polymérisation, de sorte que la RdRp polymérise un brin d'ARN complémentaire au ssARN et relargue l'oligonucléotide marqué, et
(iii) de séparation des duplex ARN formés par la RdRp enter le ssARN et le brin complémentaire polymérisé,
(iv) optionnellement, de répétition des étapes (i) à (iii) et
(v) de détection et de mesure du signal généré par le relargage de l'oligonucléotide marqué.

3. Méthode selon l'une des revendications 1 ou 2, dans laquelle la RdRp est une RdRp d'un virus de la famille des *Caliciviridae.*

4. Méthode selon l'une des revendications précédentes, dans laquelle, respectivement, le ssARN dans l'échantillon ou le modèle de ssARN ont au moins un C à leur extrémité 3'.

5. Méthode selon l'une des revendications précédentes, dans laquelle respectivement, le ssARN dans l'échantillon ou le modèle de ssARN ont un (Cn) répétitif à leur extrémité 3', avec n ≥ 3.

6. Méthode selon l'une des revendications précédentes, dans laquelle l'oligonucléotide marqué contient un marqueur fluorescent.

7. Méthode selon la revendication 6, dans laquelle l'oligonucléotide contient un marqueur fluorescent à son extrémité 5'.

8. Méthode selon l'une des revendications 6 ou 7 dans laquelle le ssARN est révélé par une molécule extinctrice de la fluorescence du marqueur fluorescent de l'oligonucléotide lorsqu'il est hybridé à l'ARN cible.

9. Méthode selon la revendication 8, dans laquelle l'oligonucléotide marqué s'hybride à l'extrémité 5' de l'ARN cible.

10. Méthode selon la revendication 6, dans laquelle un second oligonucléotide est hybridé dans l'étape (a) ou (i), respectivement au ssARN lequel second oligonucléotide est sensiblement complémentaire à une région du ssARN qui ne recouvre pas la région complémentaire de l'oligonucléotide marqué et dans laquelle le second oligonucléotide contient un groupement chimique capable d'éteindre la fluorescence du marqueur fluorescent de l'oligonucléotide marqué lorsque à la fois l'oligonucléotide marqué et le second oligonucléotide sont hybridés à la séquence de ssARN.

11. Méthode selon l'une des revendications précédentes dans laquelle l'oligonucléotide marqué et, optionnellement, le second oligonucléotide a (ont) une longueur de 5 à 20 nucléotides, de préférence de 10 à 12 nucléotides.

12. Méthode selon l'une des revendications précédentes dans laquelle le ssARN a une longueur de 16 à 40 nucléotides, de préférence de 18 à 25 nucléotides.

13. Méthode selon l'une des revendications précédentes dans laquelle le ssARN est un micro ARN ou un microARN défectueux.
